# FASCICULE DE BREVET EUROPEEN

(11) **EP 0 825 152 B1**
(45) Date de publication et mention de la délivrance du brevet: **23.01.2002**
(21) Numéro de dépôt: 97401944.0
(22) Date de dépôt: 18.08.1997
(51) Int. Cl.: C01B 39/48, C01B 39/06, B01J 29/70, B01J 29/06, C07C 5/22, C10G 45/64, C07C 6/12, C07C 2/54, C07C 2/12, C07C 5/41, C07C 4/06, C07C 5/02, C07C 5/32, C07C 41/00, B01D 53/86

(54) **Zéolithe NU-88, son procédé de préparation et ses applications catalytiques**
Zeolith NU-88, Verfahren zu seiner Herstellung sowie seine katalytische Verwendungen
Zeolite NU-88, method for its preparation and its catalytic uses

(30) Priorité: 23.08.1996 FR 9610507
(43) Date de publication de la demande: 25.02.1998
(73) Titulaire: INSTITUT FRANCAIS DU PETROLE, 92852 Rueil-Malmaison Cedex (FR)
(72) Inventeur: Casci, John Leonello, Cleveland TS23 1LB (GB); Maberly, Sheena, Middlesbrough, Cleveland (GB); Benazzi, Eric, 78360 Montesson (FR); Henney, Roland Patrick Graham, Cumbria LA12 9DR (GB); Rouleau, Loic, 69600 Oulins (FR)

(56) Documents cités:
- EP-A- 0 135 658
- EP-A- 0 463 768
- WO-A-94/00384
- WO-A-98/17581
- US-A- 4 508 837
- US-A- 4 676 958
- US-A- 4 941 963
- US-A- 5 068 096

## Description

La présente invention concerne une nouvelle zéolithe appelée ci-après zéolithe NU-88, son procédé de préparation, tout catalyseur comprenant ladite zéolithe et tout procédé catalytique utilisant ledit catalyseur.

Ainsi, selon la présente invention, la zéolithe NU-88 est caractérisée par :
-i) une composition chimique exprimée sur une base anhydre, en termes de rapports molaires d'oxydes, par la formule :

   100 XO₂, m Y₂O₃, p R_{2/n}O,

   où
   m est égal ou inférieur à 10,
   p est égal ou inférieur à 20,
   R représente un ou plusieurs cations de valence n,
   X est le silicium et/ou le germanium, de préférence le silicium,
   Y est choisi dans le groupe formé par les éléments suivants : l'aluminium, le fer, le gallium, le bore, le titane, le vanadium, le zirconium, le molybdène, l'arsenic, l'antimoine, le chrome et le manganèse, de préférence Y est l'aluminium, et
-ii) le fait qu'elle présente, sous forme brute de synthèse, un diagramme de diffraction X comportant les résultats présentés dans le tableau 1.

**Tableau 1:**

| Tableau de diffraction des rayons X de la zéolithe NU-88 (brute de synthèse) | |
|---|---|
| dₕₖₗ (10⁻¹⁰m) | l/lₘₐₓ |
| 12,1±0,35 | F ou TF (1) |
| 11,0±0,30 | F (1) |
| 9,88±0,25 | m (1) |
| 6,17±0,15 | f |
| 3,97±0,09 | TF (2) |
| 3,90±0,08 | TF (2) |
| 3,80±0,08 | f (2) |
| 3,66±0,07 | tf |
| 3,52±0,07 | tf |
| 3,27±0,07 | tf |
| 3,09±0,06 | f |
| 2,91±0,06 | f |
| 2,68±0,06 | tf |
| 2,49±0,05 | tf |
| 2,20±0,05 | tf |
| 2,059±0,05 | f |
| 1,729±0,04 | tf |

| | |
|---|---|
| (1) Ces pics ne sont pas résolus et font partie d'un même massif. | |
| (2) Ces pics ne sont pas résolus et font partie du même massif. | |

L'invention concerne aussi la zéolithe NU-88 sous forme hydrogène, désignée par H-NU-88, produite par calcination et/ou échange d'ion comme décrit ci-après. La zéolithe H-NU-88 a un diagramme de diffraction X comportant les résultats présentés dans le tableau 2.

**Tableau 2 :**

| Tableau de diffraction des rayons X de la zéolithe NU-88 (forme hydrogène) | |
|---|---|
| dₕₖₗ (10⁻¹⁰m) | l/lₘₐₓ |
| 12,1±0,35 | TF(1) |
| 11,0±0,30 | F ou TF (1) |
| 9,92±0,25 | f ou m (1) |
| 8,83±0,20 | tf |
| 6,17±0,15 | f |
| 3,99±0,10 | F ou TF (2) |
| 3,91±0,08 | TF (2) |
| 3,79±0,08 | f ou m (2) |
| 3,67±0,07 | tf |
| 3,52±0,07 | tf |
| 3,09±0,06 | f |
| 2,90±0,06 | f |
| 2,48±0,05 | f |
| 2,065±0,05 | f |
| 1,885±0,04 | tf |
| 1,733±0,04 | tf |

| | |
|---|---|
| (1) Ces pics ne sont pas résolus et font partie d'un même massif. | |
| (2) Ces pics ne sont pas résolus et font partie du même massif. | |

Ces diagrammes sont obtenus à l'aide d'un diffractomètre en utilisant la méthode classique des poudres avec le rayonnement Kα du cuivre Cu K alpha. A partir de la position des pics de diffraction représentée par l'angle 2θ, on calcule, par la relation de Bragg, les équidistances réticulaires dₕₖₗ caractéristiques de l'échantillon. Le calcul de l'intensité se fait sur la base d'une échelle d'intensité relative sur laquelle on attribue une valeur de 100 à la raie présentant l'intensité la plus forte sur le diagramme de diffraction X, et alors :
très faible (tf) signifie inférieure à 10,
faible (f) signifie inférieure à 20,
moyenne (m) signifie comprise entre 20 et 40,
forte (F) signifie comprise entre 40 et 60,
très forte (TF) signifie supérieure à 60.

Les diffractogrammes X à partir desquels ces données ont été obtenues (espacement d et intensités relatives) sont caractérisés par de larges réflexions avec de nombreux pics formant des épaulements sur d'autres pics d'intensité supérieure. Il peut arriver que certains épaulements, ou tous les épaulements, ne soient pas résolus. Ceci peut se produire pour des échantillons faiblement cristallins ou des échantillons au sein desquels les cristaux sont suffisamment petits pour donner un élargissement significatif des rayons X. Cela peut également être le cas lorsque l'équipement ou les conditions mis en oeuvre pour obtenir le diagramme diffèrent de ceux utilisés ici.

On estime que la zéolithe Nu-88 possède une nouvelle structure de base ou topologie qui est caractérisée par son diagramme de diffraction X. La zéolithe NU-88 sous sa "forme brute de synthèse" possède sensiblement les caractéristiques obtenues par diffraction X, présentées dans le tableau 1, et se distingue ainsi des zéolithes connues. L'objet de l'invention comprend aussi toute zéolithe de même type structural que celui de la zéolithe Nu-88.

Les tableaux 1 et 2 et les diffractogrammes des figures i et 2 sont relativement peu habituels pour des structures zéolithiques. Par conséquent, ces données semblent indiquer que la zéolithe NU-88 présente une structure à défaut.

Dans le cadre de la définition de la composition chimique donnée ci-dessus, m est généralement compris entre 0,1 et 10, de préférence entre 0,2 et 9, et de façon encore plus préférée entre 0,6 et 8 ; il apparaît que la zéolithe NU-88 s'obtient généralement le plus aisément sous une forme très pure lorsque m est compris entre 0,6 et 8. p est compris entre 0 et 3, 92.

Cette définition englobe également la zéolithe NU-88 sous sa "forme brute de synthèse", ainsi que des formes obtenues par déshydratation et/ou calcination et/ou échange d'ions. L'expression "sous sa forme brute de synthèse" désigne le produit obtenu par synthèse et par lavage avec ou sans séchage ou déshydratation. Sous sa "forme brute de synthèse", la zéolithe NU-88 peut comporter un cation de métal M, qui est un alcalin , notamment du sodium, et/ou de l'ammonium, et elle peut comporter des cations organiques azotés tels que ceux décrits ci-après ou leurs produits de décomposition, ou encore leurs précurseurs. Ces cations organiques azotés sont désignés ici par la lettre Q, qui inclut aussi les produits de décomposition et les précurseurs desdits cations organiques azotés.

Ainsi, la zéolithe Nu-88, sous sa forme "brute de synthèse" (non calcinée), est caractérisée par :
i) une composition chimique exprimée sur une base anhydre, en termes de rapports molaires d'oxydes :
   100 XO₂ : inférieur ou égal à 10 Y₂O₃ : inférieur ou égal à 10 Q : inférieur ou égal à 10 M₂O,
      où
   X est le silicium et/ou le germanium,
   Y est choisi dans le groupe formé par les éléments suivants : l'aluminium, le fer, le gallium, le bore, le titane, le vanadium, le zirconium, le molybdène, l'arsenic, l'antimoine, le chrome et le manganèse,
   M est au moins un cation de métal alcalin (groupe lA de la Classification Périodique des Eléments) et/ou l'ammonium, et
   Q est au moins un cation organique azoté ou un précurseur de cation organique azoté ou un produit de décomposition de cation organique azoté.
ii) le fait qu'elle présente, sous forme brute de synthèse, un diagramme de diffraction X comportant les résultats présentés dans le tableau 1

Les compositions indiquées ci-dessus pour la zéolithe NU-88 sont données sur une base anhydre, bien que la zéolithe NU-88 sous sa "forme brute de synthèse" et les formes activées de la zéolithe NU-88, c'est-à-dire résultant d'une calcination et/ou d'un échange d'ions, puissent contenir de l'eau. La teneur molaire en H₂O de telles formes, y compris la zéolithe NU-88 sous sa "forme brute de synthèse", varie selon les conditions dans lesquelles elles ont été préparées et conservées après synthèse ou activation. Les quantités molaires d'eau contenue dans ces formes sont typiquement comprises entre 0 et 100 % XO₂.

Les formes calcinées de la zéolithe NU-88 ne contiennent pas de composé organique azoté, ou en quantité moindre que la "forme brute de synthèse", dans la mesure où la substance organique est éliminée en majeure partie, généralement par un traitement thermique consistant à brûler la substance organique en présence d'air, l'ion hydrogène (H⁺) formant alors l'autre cation.

Ainsi la zéolithe Nu-88, sous sa forme hydrogène, est caractérisée par :
i) une composition chimique suivante exprimée sur une base anhydre, en termes de rapports molaires d'oxydes :
   100 XO₂ : inférieur ou égal à 10 Y₂O₃ : inférieur ou égal à 10 M₂O,
      où
   X est le silicium et/ou le germanium,
   Y est choisi dans le groupe formé par les éléments suivants : l'aluminium, le fer, le gallium, le bore, le titane, le vanadium, le zirconium, le molybdène, l'arsenic, l'antimoine, le chrome et le manganèse, et
   M est au moins un cation de métal alcalin (groupe lA de la Classification Périodique des Eléments) et/ou l'ammonium et/ou l'hydrogène,
ii) le fait qu'elle présente, sous forme brute de synthèse, un diagramme de diffraction X comportant les résultats présentés dans le tableau 2.

Parmi les formes de zéolithe NU-88 obtenues par échange d'ions, la forme ammonium (NH₄⁺) est importante car elle peut être facilement convertie sous la forme hydrogène par calcination. La forme hydrogène et les formes contenant des métaux introduits par échange d'ions sont décrites ci-dessous. Dans certains cas, le fait de soumettre la zéolithe selon l'invention à l'action d'un acide peut donner lieu à l'élimination partielle ou totale d'un élément de base tel que l'aluminium, ainsi que la génération de forme hydrogène. Ceci peut constituer un moyen de modifier la composition de la substance de la zéolithe après qu'elle a été synthétisée.

L'invention permet également d'obtenir la zéolithe NU-88 sous forme hydrogène, appelée H-NU-88, produite par calcination et par échange d'ions comme décrit ci-après.

Un des objets de l'invention est donc la zéolithe Nu-88 au moins en partie sous forme H⁺ (telle que définie ci-dessus) ou NH4+ ou métallique, ledit métal étant choisi dans le groupe formé par les groupes IA, IB, IIA, IIB, IIIA, IIIB (y compris les terres rares), VIII,

Sn, Pb et Si, de préférence au moins en partie sous forme H+ ou au moins en partie sous forme métallique. Une telle zéolithe présente généralement un diagramme de diffraction X comportant les résultats présentés dans le tableau 1.

Quelques modes de réalisation de la zéolithe NU-88 sont exposés dans les exemples. Globalement, on fait réagir un mélange aqueux comportant au moins une source d'au moins un oxyde XO₂, au moins une source d'au moins un oxyde Y₂O₃, éventuellement au moins une source d'au moins un oxyde M₂O et au moins un cation organique Q azoté, ou un précurseur de cation organique azoté ou un produit de décomposition de cation organique azoté, le mélange présentant généralement la composition molaire suivante:
XO₂/Y₂O₃ au moins 10, de préférence de 10 à 60, de préférence encore de 15 à 50
(R1/n)OH/XO₂ de 0,01 à 2, de préférence de 0,05 à 1, de préférence encore de 0,10 à 0,75
H₂O/XO₂ de 1 à 500, de préférence de 5 à 250, de préférence encore de 25 à 75
Q/XO₂ de 0,005 à 1, de préférence de 0,02 à 1, de préférence encore de 0,05 à 0,5
LₚZ/XO₂ de 0 à 5, de préférence de 0 à 1, de préférence encore de 0 à 0,25,
   où X est le silicium et/ou le germanium, de préférence le silicium,
Y est choisi dans le groupe formé par les éléments suivants : aluminium, fer, bore, titane, vanadium, zirconium, molybdène, arsenic, antimoine, gallium, chrome, manganèse, de préférence Y est l'aluminium,
R est un cation de valence n qui comporte M (un cation de métal alcalin et/ou de l'ammonium), et/ou Q (un cation organique azoté ou un précurseur de celui-ci ou un produit de décomposition de celui-ci)
LₚZ est un sel, Z étant un anion de valence p et L un ion de métal alcalin ou ammonium qui peut être similaire à M ou un mélange de M et d'un autre ion de métal alcalin ou un ion ammonium nécessaire pour équilibrer l'anion Z, Z pouvant comporter un radical acide ajouté par exemple sous la forme d'un sel de L ou d'un sel d'aluminium.

Dans certains cas, il peut être avantageux d'ajouter un sel LₚZ. Ainsi un procédé de préparation préféré est tel que le milieu aqueux comprend dudit sel. On peut citer à titre d'exemple pour Z des radicaux acides forts tels que du bromure, du chlorure, de l'iodure, du sulphate, du phosphate ou du nitrate, ou des radicaux acides faibles tels que les radicaux acides organiques, par exemple du citrate ou de l'acétate. Bien que le LpZ ne soit pas essentiel, il peut accélérer la cristallisation de la zéolithe NU-88 à partir du mélange réactionnel et il peut également affecter la taille et la forme des cristaux constituant la zéolithe NU-88. Dans tous les cas, la réaction se poursuit jusqu'à obtention de la cristallisation.
De nombreuses zéolithes ont été préparées avec des cations organiques azotés, et des noyaux hétérocycliques ont notamment été largement utilisés pour la synthèse de la zéolithe. La zéolithe ZSM-12 a été synthétisée en utilisant différentes formes hétérocycliques, par exemple la structure à base de pyrolidinium 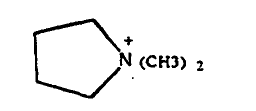 et la structure à base de pipéridinium 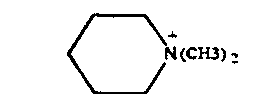 a été décrite par Rosinski et al dans le brevet américain US-A-4.391.785 (Mobil).

La synthèse de la zéolithe TPZ-12, qui est un isomère de structure de la zéolithe ZSM-12, est décrite par Teijin dans la demande de brevet européen EP-A-135.658. Les structures considérées comme étant adaptées à la synthèse de la zéolithe TPZ-12 pure sont celle à base de bispyrrolidinium 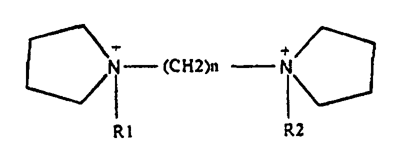 et celle à base de bispiperidinium 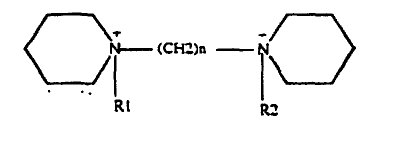 où on a fait varier n de 4 à 6, et R₁ et R₂ : H, méthyl, éthyl, propyl ou butyl.

La synthèse de la zéolithe ZSM-12 a également été réalisée avec des structures comportant plus d'un noyau hétérocyclique. L'utilisation du composé du type polyméthonium 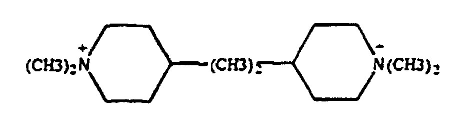 est décrite par Valyocsik dans le brevet américain US-A-4.539.193 (Mobil). L'utilisation de structures à base de bis (butyl pyrrolidinium) de formule générale 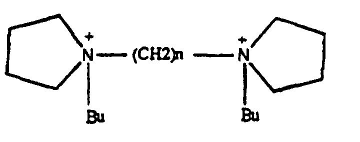 est décrite par Valyocsik dans le brevet américain US-A-4.941.963 (Mobil). Quatre structures ont été testées en faisant varier n de 4 à 7. La synthèse de la zéolithe ZSM-11 pure a seulement été réalisée avec n égal à 6 ou à 7.

Selon l'invention, de préférence, Q est un cation de bis(méthyl pyrrolidinium), ou un de ses produits de décomposition ou un de ses précurseurs, de formule générale 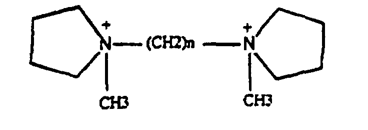 avec n = 4, 5 ou 6, ou Q est le butane-1,4-bis(bisquinuclidinium) bromure, ou un de ses produits de décomposition ou un de ses précurseurs, de formule chimique suivante: 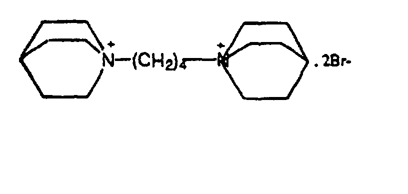 par exemple, Q est l'hexane-1,6-bis(méthylpyrrolidinium) bromure, le pentane-1,5-bis(méthylpyrrolidinium) bromure, le butane-1,4-bis(méthylpyrrolidinium) bromure, ou bien Q est le butane-1,4-bis(quinuclidinium) bromure .

M et/ou Q peuvent être ajoutés sous forme d'hydroxydes ou de sels d'acides minéraux à condition que le rapport (R_{1/n})OH/XO₂ soit respecté.

De telles substances peuvent être employées sous forme de mélanges simples, ou elles peuvent être préchauffées ensemble dans le réacteur, de préférence en solution, avant de leur ajouter les autres réactifs nécessaires à la synthèse de la zéolithe NU-88.

Le cation M utilisé de préférence est un métal alcalin, notamment du sodium, XO₂ étant de préférence de la silice (SiO₂) et l'oxyde Y₂O₃ étant de préférence de l'alumine (Al₂O₃).

Dans le cas préféré où X est le silicium, la source de silice peut être l'une quelconque de celles couramment utilisées dans la synthèse des zéolithes, par exemple de la silice solide en poudre, de l'acide silicique, de la silice colloïdale ou de la silice dissoute. Parmi les silices en poudre, on peut utiliser les silices précipitées, notamment celles obtenues par précipitation à partir d'une solution de silicate de métal alcalin, telle que celle appelée "KS 300" fabriquée par AKZO, et des produits similaires, des silices aérosiles, des silices pyrogénées, par exemple du "CAB-O-SIL" et des gels de silice dans des teneurs appropriées pour être utilisées dans les pigments de renforçage (en anglais "reinforcing") destinés au caoutchouc et au caoutchouc siliconé. On peut utiliser des silices colloïdales présentant différentes tailles de particules, par exemple de diamètre équivalent moyen compris entre 10 et 15 µm ou entre 40 et 50 µm telles que celles commercialisées sous les marques déposées "LUDOX", "NALCOAG" et "SYTON". Les silices dissoutes qui peuvent être employées comprennent également les silicates au verre soluble disponibles dans le commerce, contenant 0,5 à 6,0, notamment 2,0 à 4,0 moles de SiO₂ par mole d'oxyde de métal alcalin, des silicates de métaux alcalins "actifs" tels que définis dans le brevet britannique GB-A-1.193.254, et des silicates obtenus par dissolution de silice dans un hydroxyde de métal alcalin ou un hydroxyde d'ammonium quaternaire, ou encore un mélange de ceux-ci.

Dans le cas préféré où Y est l'aluminium, la source d'alumine est de préférence de l'aluminate de sodium, ou un sel d'aluminium, par exemple du chlorure, du nitrate ou du sulfate, un alkoxyde d'aluminium ou de l'alumine proprement dite, de préférence sous forme hydratée ou hydratable, comme par exemple de l'alumine colloïdale, de la pseudoboehmite, de l'alumine gamma ou du trihydrate alpha ou bêta. On peut également utiliser des mélanges des sources citées ci-dessus.

Certaines ou l'ensemble des sources d'alumine et de silice peuvent éventuellement être ajoutées sous forme d'aluminosilicate.

On fait généralement réagir le mélange réactionnel sous une pression de réaction autogène, éventuellement en ajoutant du gaz, par exemple de l'azote, à une température comprise entre 85°C et 200°C, de préférence entre 120°C et 180°C, et de manière encore préférentielle à une température qui ne dépasse pas 165°C, jusqu'à la formation des cristaux de zéolithe NU-88, ce temps variant généralement entre 1 heure et plusieurs mois en fonction de la composition du réactif et de la température de service. La mise en réaction peut s'effectuer sous agitation, ce qui est préférable dans la mesure où le temps de réaction s'en trouve réduit et la pureté du produit améliorée.

Il peut être avantageux d'utiliser des germes afin de réduire le temps nécessaire à la formation des cristaux et/ou la durée totale de cristallisation. Il peut également être avantageux de favoriser la formation de la zéolithe NU-88. De tels germes comprennent des zéolithes, notamment des cristaux de zéolithe NU-88. Les germes cristallins sont généralement ajoutés dans une proportion comprise entre 0,01 et 10 % du poids de silice utilisée dans le mélange réactionnel.

A la fin de la réaction, la phase solide est recueillie dans un filtre et lavée; elle est ensuite prête pour des étapes ultérieures telles que le séchage, la déshydratation et la calcination et/ou l'échange d'ions.

Si le produit de la réaction contient des ions de métaux alcalins, ceux-ci doivent être éliminés au moins en partie en vue de la préparation de la forme hydrogène de la zéolithe NU-88, au moyen d'au moins un échange d'ions avec un acide, notamment un acide minéral tel que l'acide chlorhydrique et/ou à l'aide du composé d'ammonium obtenu par échange d'ions avec une solution d'un sel d'ammonium tel que du chlorure d'ammonium. L'échange d'ions peut être effectué au moyen d'une mise en suspension épaisse, à une ou plusieurs reprises, dans la solution d'échange d'ions. La zéolithe est généralement calcinée avant l'échange d'ions afin d'éliminer toute substance organique absorbée dans la mesure où l'échange d'ions s'en trouve facilité. Toutes les conditions opératoires d'un tel échange ionique sont connues de l'homme du métier.

D'une manière générale, on peut remplacer le(s) cation(s) de la zéolithe NU-88 par tout cation ou tous cations de métaux, notamment ceux des groupes IA, IB, IIA, IIB, IIIA, IIIB (y compris les terres rares) et VIII (y compris les métaux nobles) de la classification périodique des éléments, et par l'étain, le plomb et le bismuth. L'échange s'effectue normalement avec une solution contenant un sel du cation approprié, de la manière connue de l'homme du métier.

L'invention concerne également la composition d'un catalyseur contenant la zéolithe NU-88 Ainsi un des objets de l'invention est un catalyseur comprenant une zéolithe Nu-88 telle que décrite précédemment ou préparée selon le procédé de préparation décrit précédemment. L'invention concerne également un catalyseur qui comprend une telle zéolithe Nu-88, et qui comprend en outre au moins un liant ou au moins un support ou au moins une autre zéolithe ou au moins un métal choisi dans le groupe formé par les éléments Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Re et Rh.

Dans les catalyseurs selon l'invention, XO₂ est de préférence de la silice et Y₂O₃ est de préférence de l'alumine. De tels catalyseurs peuvent être utilisés dans un grand nombre de procédés catalytiques et avec une large gamme de charges d'alimentation.

Les formes de zéolithe NU-88 utiles en catalyse comprennent généralement les formes hydrogène et ammonium préparées selon les méthodes décrites ci-avant. Mais les catalyseurs selon l'invention comportant de la zéolithe NU-88 peuvent également contenir un ou plusieurs éléments, notamment des métaux ou leurs cations, ou des composés de ces éléments, notamment des oxydes de métaux. Ces catalyseurs peuvent être préparés par échange d'ions ou par imprégnation de la zéolithe NU-88 avec ledit élément, cation ou composé, ou avec un précurseur approprié dudit cation ou composé. Un tel échange d'ions ou une telle imprégnation peuvent être réalisés sur la zéolithe NU-88 au moins en partie, de préférence pratiquement totalement sous sa "forme brute de synthèse", calcinée ou non, sous forme hydrogène et/ou sous forme ammonium et/ou sous toute autre forme échangée (métallique ou non).

Dans les cas où une forme de zéolithe NU-88 contenant un métal est préparée par échange d'ions, il peut être souhaitable d'effectuer un échange complet dudit métal, ce qui signifie que sensiblement l'ensemble des sites échangeables est occupé par ledit métal. De telles formes peuvent être particulièrement utiles dans des procédés de séparation. Dans la plupart des cas cependant, il est préférable de n'effectuer qu'un échange partiel du métal, les sites restants étant occupés par un autre cation, notamment les cations hydrogène ou ammonium. Dans certains cas, il peut être souhaitable d'introduire deux cations métalliques ou plus par échange d'ions.

Dans les cas où la zéolithe NU-88 est imprégnée avec un composé métallique pour former un catalyseur, le composé métallique peut être ajouté dans une proportion appropriée, mais une proportion maximum de 20 % en poids est généralement suffisante pour la plupart des applications; pour certaines applications, on ne dépasse généralement pas 10 % en poids, et des quantités allant jusqu'à 5 % sont souvent appropriées. L'imprégnation peut être effectuée par toute méthode appropriée connue dans le cadre de la préparation des catalyseurs.

Les formes à échange de métaux ou les formes dans lesquelles un composé métallique a été imprégné peuvent être utilisées telles quelles ou traitées en vue de produire un dérivé actif. Les traitements comprennent la réduction, par exemple dans une atmosphère comportant de l'hydrogène, afin de produire un métal ou d'autres formes réduites. Ces traitements peuvent être réalisés à un stade approprié de la préparation du catalyseur ou ils peuvent également être aisément réalisés dans le réacteur catalytique.

Les compositions catalytiques comprenant la zéolithe NU-88 peuvent être associées, si on le souhaite, à une matrice minérale qui peut être soit inerte, soit active sur le plan catalytique. La matrice peut être utilisée seulement comme liant pour maintenir les particules de zéolithe ensemble, éventuellement sous une forme particulière, par exemple sous forme de pastille ou de produit d'extrusion, ou bien elle peut fonctionner comme un diluant inerte, par exemple pour contrôler l'activité par unité de poids de catalyseur. Lorsque la matrice minérale ou le diluant présentent eux-mêmes une activité catalytique, ils peuvent former ainsi une partie efficace de la composition catalytique zéolithe-matrice. Les matrices minérales et les diluants appropriés comprennent des substances utilisées de manière classique comme supports de catalyseur, telles que la silice, les différentes formes d'alumine, des argiles telles que les bentonites, les montmorillonites, la sépiolite, l'attapulgite, de la terre à foulon et des matières poreuses synthétiques telles que silice-alumine, silice-zircone, silicethorine, silice-glucine ou silice-dioxyde de titane. Des combinaisons de matrices peuvent être envisagées dans le cadre de la présente invention, notamment des combinaisons de matrices inertes et de matrices présentant une activité catalytique.

Lorsque la zéolithe NU-88 est associée à une substance matricielle minérale ou à une pluralité de telles substances, la proportion de substance(s) matricielle(s) dans la composition totale s'élève généralement jusqu'à environ 90 % en poids, de préférence jusqu'à 50 % en poids et de manière encore préférentielle jusqu'à 30 % en poids.

Pour certaines applications, on peut utiliser une autre zéolithe ou tamis moléculaire conjointement avec la zéolithe NU-88 pour former un catalyseur. Une telle combinaison peut être employée telle quelle ou associée à l'une ou plusieurs des substances matricielles décrites ci-avant. On peut citer, à titre d'exemple particulier de la mise en oeuvre d'une telle composition, son utilisation comme additif de catalyseur pour craquage catalytique fluide, auquel cas la zéolithe NU-88 est de préférence utilisée dans une proportion de 0,5 à 5 % en poids de catalyseur total.

Pour d'autres applications, la zéolithe NU-88 peut être combinée avec un autre catalyseur tel que du platine sur de l'alumine.

Toute méthode appropriée pour mélanger la zéolithe NU-88 avec une matrice organique et/ou une autre zéolithe peut être mise en oeuvre, notamment celle adaptée à la forme finale sous laquelle le catalyseur est utilisé, par exemple produit d'extrusion, pastille ou granulé.

Si l'on utilise des zéolithes NU-88 pour former un catalyseur conjointement avec un composant métallique (par exemple un composant d'hydrogénation/ déshydrogénation ou un autre métal ayant une activité catalytique) en plus de la matrice minérale, le composant métallique peut être échangé ou imprégné dans la zéolithe NU-88 elle-même avant d'ajouter la substance matricielle, ou dans la composition zéolithe-matrice. Pour certaines applications, il peut être avantageux d'ajouter le composant métallique à la totalité ou à une partie de la substance matricielle avant de mélanger cette dernière avec la zéolithe NU-88.

Une large gamme de catalyseurs de conversion d'hydrocarbures comportant la zéolithe NU-88 peut être préparée par échange d'ions ou par imprégnation de la zéolithe avec un ou plusieurs cations ou oxydes dérivés d'éléments parmi lesquels : Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Re et Rh.

Au cas où les catalyseurs comportant la zéolithe NU-88 contiennent un ou plusieurs composants d'hydrogénation/déshydrogénation, tels que les métaux Ni, Co, Pt, Pd, Re et Rh, ces composants peuvent être introduits par échange d'ions ou par imprégnation d'un composé approprié de ce métal.

Les compositions catalytiques comportant la zéolithe NU-88 peuvent trouver leur application dans des réactions comprenant des hydrocarbures aliphatiques saturés et insaturés, des hydrocarbures aromatiques, des composés organiques oxygénés et des composés organiques contenant de l'azote et/ou du soufre, ainsi que des composés organiques contenant d'autres groupes fonctionnels.

Un des objets de l'invention concerne donc également tout procédé catalytique tel que la zéolithe NU-88 est comprise dans le catalyseur. D'une manière générale, les compositions de catalyseurs contenant la zéolithe NU-88 peuvent être utilisées efficacement pour réaliser des réactions d'isomérisation, de transalkylation et de dismutation, d'alkylation et de désalkylation, de déshydratation et d'hydratation, d'oligomérisation et de polymérisation, de cyclisation, d'aromatisation, de craquage, d'hydrogénation et de déshydrogénation, d'oxydation, d'halogénation, de synthèse d'amines, d'hydrodésulfuration et d'hydrodénitrification, d'élimination catalytique des oxydes d'azote (soit, de préférence, par réduction, généralement par des composés azotés ou par des hydrocarbures, soit par décomposition), la formation d'éther et la conversion d'hydrocarbures et la synthèse de composés organiques en général.

Les procédés mentionnés ci-dessus peuvent être effectués soit en phase liquide, soit en phase vapeur, dans des conditions choisies pour être les plus appropriées pour chaque réaction individuelle. Par exemple, les réactions effectuées en phase vapeur peuvent comporter la mise en oeuvre d'opérations à lit fluide, à lit fixe ou à lit mobile. Des diluants de traitement peuvent être utilisés si nécessaire. Selon le procédé mis en oeuvre, les diluants appropriés peuvent être des gaz inertes (tels que de l'azote ou de l'hélium), des hydrocarbures, du dioxyde de carbone, de l'eau ou de l'hydrogène. Le diluant peut être inerte ou bien il peut exercer une action chimique. Il peut être avantageux, notamment si l'on utilise de l'hydrogène, d'inclure un composant métallique, tel qu'un composant d'hydrogénation/ déshydrogénation, par exemple ou ou plusieurs des métaux Ni, Co, Pt, Pd, Re ou Rh dans la composition du catalyseur.

La présente invention concerne également tout procédé de conversion d'hydrocarbures au cours duquel on met en contact un alkylbenzène ou un mélange d'alkylbenzènes dans des conditions d'isomérisation, en phase vapeur ou liquide, avec un catalyseur contenant de la zéolithe NU-88.

Les réactions d'isomérisation pour lesquelles les catalyseurs contenant de la zéolithe NU-88 sont particulièrement utiles sont celles comportant des alcanes et des molécules aromatiques substituées, notamment des xylènes. Ces réactions peuvent comprendre celles qui peuvent être effectuées en présence d'hydrogène. Les compositions de catalyseurs contenant de la zéolithe NU-88 qui sont particulièrement utiles dans les réactions d'isomérisation comprennent celles dans lesquelles la zéolithe NU-88 se présente sous sa forme acide (H⁺), sous la forme obtenue après échange de cations, sous sa forme contenant des métaux ou dans des combinaisons des formes précitées. Les formes dans lesquelles le métal est un composant d'hydrogénation/déshydrogénation tel que Ni, Co, Pt, Pd, Re ou Rh s'avèrent particulièrement utiles.

Les réactions d'isomérisation particulières dans lesquelles un catalyseur contenant de la zéolithe NU-88 peut se révéler utile comprennent l'isomérisation et l'hydro-isomérisation de xylènes, ou de paraffines, notamment des hydrocarbures normaux de C₄ à C₁₀, ou l'isomérisation d'oléfines et le déparaffinage catalytique.

L'isomérisation et l'hydro-isomérisation du xylène peuvent être effectuées en phase liquide ou en phase vapeur. En phase liquide, les conditions d'isomérisation appropriées comportent une température comprise entre 0 et 350°C, une pression comprise entre 0,1 et 20 MPa (absolus), de préférence entre 0,5 et 7 MPa (absolus), et dans le cas de la mise en oeuvre d'un régime dynamique, un poids de catalyseur par poids par heure (PPH) de préférence compris entre 1 et 30 h⁻¹ sur la base de la composition totale du catalyseur. Un diluant peut éventuellement être présent, de préférence l'un ou plusieurs de ceux présentant une température critique supérieure aux conditions d'isomérisation mises en oeuvre. Le diluant, si l'on en utilise un, peut comporter de 1 à 90 % en poids de la charge. Les réactions d'isomérisation et d'hydro-isomérisation du xylène en phase vapeur sont effectuées à une température comprise de manière appropriée entre 100 et 600°C, de préférence entre 200 et 500°C, à une pression comprise entre 0,05 et 10 MPa (absolus), de préférence entre 0,1 et 5 MPa (absolus), et à une valeur de poids de catalyseur par poids par heure (PPH) pouvant aller jusqu'à 80 sur la base de la composition totale du catalyseur.

Lorsque l'isomérisation du xylène est réalisée en présence d'hydrogène (en phase vapeur), le composant d'hydrogénation/déshydrogénation utilisé de préférence est le Pt ou le Ni. Le composant d'hydrogénation/déshydrogénation est généralement ajouté dans une proportion comprise entre 0,5 et 2 % en poids total de catalyseur. Des métaux et/ou des oxydes métalliques supplémentaires peuvent être présents dans la composition du catalyseur.

Dans l'isomérisation du xylène, de l'éthylbenzène peut être présent dans la charge de xylène dans une proportion pouvant atteindre 40 % en poids. Avec des compositions de catalyseur comportant de la zéolithe NU-88, l'éthylbenzène subit généralement une transalkylation avec lui-même et avec les xylènes pour former des composés aromatiques plus lourds et plus légers. L'éthylbenzène réagit généralement également pour former du benzène et du gaz léger, notamment à des températures supérieures à 400°C. Avec de telles charges de xylène contenant de l'éthylbenzène, lorsque la réaction est conduite en présence d'hydrogène avec une composition catalytique comprenant de la zéolithe NU-88 et un composant d'hydrogénation/déshydrogénation, une certaine partie de l'éthylbenzène est transformée par isomérisation en xylènes. Il peut également être avantageux de conduire les réactions d'isomérisation de xylène en présence d'un composé hydrocarboné, notamment une paraffine ou un naphtène, avec ou sans la présence complémentaire d'hydrogène. L'hydrocarbure semble améliorer les performances du catalyseur dans la mesure où les réactions qui entraînent la perte de xylènes sont supprimées et, notamment lorsque les réactions sont conduites en l'absence d'hydrogène, la vie du catalyseur est augmentée.

La présente invention concerne en outre un procédé de conversion d'hydrocarbures dans lequel on met en contact un ou plusieurs composés aromatiques alkylés dans des conditions de transalkylation, en phase vapeur ou en phase liquide, avec un catalyseur contenant de la zéolithe NU-88. Les catalyseurs contenant de la zéolithe NU-88 sont particulièrement utiles dans les réactions de transalkylation et/ou de dismutation, notamment dans les réactions impliquant des molécules aromatiques mono-, di-, tri- et tétrasubstituées par l'alkyle, notamment le toluène et les xylènes.

Les compositions de catalyseurs contenant de la zéolithe NU-88 qui s'avèrent particulièrement utiles dans le cadre de réactions de transalkylation et/ou de dismutation incluent les compositions dans lesquelles le composant NU-88 se présente sous sa forme acide (H⁺), sa forme obtenue par échange de cations, ou d'autres formes contenant des métaux ou des combinaisons de ces différentes formes. La forme acide et les formes dans lesquelles le métal est un composant d'hydrogénation/déshydrogénation tel que Ni, Co, Pt, Pd, Re ou Rh sont particulièrement efficaces.

On peut citer comme exemple particulier de procédés importants la dismutation du toluène et la réaction du toluène avec des composés aromatiques comportant au moins 9 atomes de carbone par molécule, par exemple les triméthylbenzènes.

La dismutation du toluène peut être conduite en phase vapeur, en présence ou en l'absence d'hydrogène, bien qu'il soit préférable d'opérer en présence d'hydrogène dans la mesure où celui-ci contribue à supprimer la désactivation du catalyseur. Les conditions de réaction les plus favorables sont les suivantes : températures comprises entre 250 et 650°C, de préférence entre 300 et 550°C; pressions comprises entre 0,03 et 10 MPa (absolus), de préférence entre 0,1 et 5 MPa (absolus); poids par poids par heure (PPH) jusqu'à 50 (sur la base de la composition totale du catalyseur).

Lorsque la dismutation du toluène est conduite en présence d'hydrogène, le catalyseur peut éventuellement contenir un composant d'hydrogénation/ déshydrogénation. On utilisera de préférence un composant d'hydrogénation/ déshydrogénation tel que Pt, Pd ou Ni. Le composant d'hydrogénation/ déshydrogénation est normalement ajouté à une concentration pouvant atteindre 5 % en poids de la composition totale du catalyseur. Des métaux et/ou des oxydes métalliques complémentaires peuvent être présents dans la composition du catalyseur, par exemple jusqu'à 5 % en poids de la composition totale du catalyseur.

La présente invention concerne également un procédé de conversion d'hydrocarbures dans lequel on met en contact un composé oléfinique ou aromatique avec un composé alkylant approprié dans des conditions d'alkylation, en phase vapeur ou en phase liquide, avec un catalyseur contenant de la zéolithe NU-88.

Parmi les réactions d'alkylation pour lesquelles les catalyseurs contenant de la zéolithe NU-88 sont particulièrement utiles, on peut citer l'alkylation du benzène ou de molécules aromatiques substituées avec du méthanol ou une oléfine ou de l'éther. Parmi les exemples spécifiques de tels procédés, on citera la méthylation du toluène, la synthèse de l'éthylbenzène et la formation de l'éthyltoluène et du cumène. Les catalyseurs d'alkylation utilisés dans les procédés selon ce mode de réalisation de l'invention peuvent comporter d'autres substances, notamment des oxydes métalliques qui peuvent améliorer les performances catalytiques.

Grâce à l'utilisation d'un catalyseur contenant de la zéolithe NU-88, des hydrocarbures peuvent être produits en conduisant des réactions d'oligomérisation, de cyclisation et/ou d'aromatisation sur des composés insaturés tels que l'éthène, le propène ou le butène, ou sur des composés saturés tels que le propane ou le butane ou sur des mélanges d'hydrocarbures tels que des naphtas légers. Pour certaines réactions, notamment les réactions d'aromatisation, il peut être utile que le catalyseur contienne un métal ou un oxyde métallique, notamment du platine, du gallium, du zinc ou leurs oxydes.

Les catalyseurs contenant de la zéolithe NU-88 sont utiles pour une large gamme de réactions de craquage, parmi lesquelles le craquage d'oléfines, de paraffines ou d'aromatiques, ou encore de leurs mélanges. L'utilisation de la zéolithe NU-88 comme additif de catalyseur de craquage catalytique fluide est particulièrement utile pour améliorer le produit de la réaction de craquage. La zéolithe NU-88 peut également être utilisée comme composant d'un catalyseur pour le déparaffinage catalytique dans le cadre de procédés d'hydrocraquage.

Les procédés d'hydrogénation et/ou de déshydrogénation, par exemple la déshydrogénation d'alcanes en oléfines correspondantes, sont conduits de manière efficace en mettant en contact la charge appropriée, dans des conditions adaptées, avec un catalyseur contenant de la zéolithe NU-88, notamment lorsque cette dernière comporte aussi un composant d'hydrogénation/déshydrogénation tel que Ni, Co, Pt, Pd, Re ou Ru.

Un catalyseur contenant de la zéolithe NU-88 est également un catalyseur utile pour la formation d'éthers, notamment par la réaction de deux alcools ou par la réaction d'une oléfine avec un alcool.

L'invention est illustrée au moyen des exemples suivants.

### Exemple 1 : Synthèse de zéolithe NU-88 avec de l'hexane-1,6-bis (méthylpyrrolidinium) bromure (HexPyrr)

La structure de l'hexane-1,6-bis(méthylpyrrolidinium) bromure (HexPyrr) est la suivante : 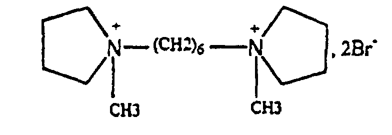 Un mélange réactionnel de composition molaire

60 SiO₂ : 2 Al₂O₃ : 10 Na₂O : 10 HexPyrr : 3000 H₂O

a été préparé à partir de :
48,07 g de "CAB-O-SIL" (BDH Ltd)
12,303 g de solution SoAl 235 (Laroche)
   (composition en % poids : 22,10 % Al₂O₃; 20,40 % Na₂O; 57,50 % H₂O)
7,4 g de pastilles d'hydroxyde de sodium
57,2 g de HexPyrr (composition en % poids : 96,50 % HexPyrr; 3,50 % H₂O)
709 g d'eau.

Le mélange a été préparé selon le mode opératoire suivant :
A - solution contenant l'hydroxyde de sodium et de l'aluminate de sodium dans de l'eau (approximativement 200 g)
B - solution contenant l'HexPyrr dans de l'eau (approximativement 150 g)
C - dispersion du CAB-O-SIL dans l'eau restante.

La solution A a été ajoutée à la dispersion C sous agitation; la solution B a ensuite été ajoutée. L'agitation a été poursuivie jusqu'à obtention d'un gel homogène. Le mélange obtenu a ensuite été transféré dans un autoclave en acier inoxydable d'une capacité de 1 litre. Le mélange a été porté à une température de 160°C. Cette température a été maintenue pendant toute la durée de la réaction. De plus, le mélange a été maintenu sous agitation à l'aide d'un agitateur à palettes inclinées.

Des échantillons du mélange réactionnel ont été régulièrement prélevés et le déroulement de la réaction a été surveillé par l'intermédiaire du pH. Après 13 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'émission atomique. On a trouvé la composition molaire suivante :

100 SiO₂ : 4,82 Al₂O₃ : 0,337 Na₂O.

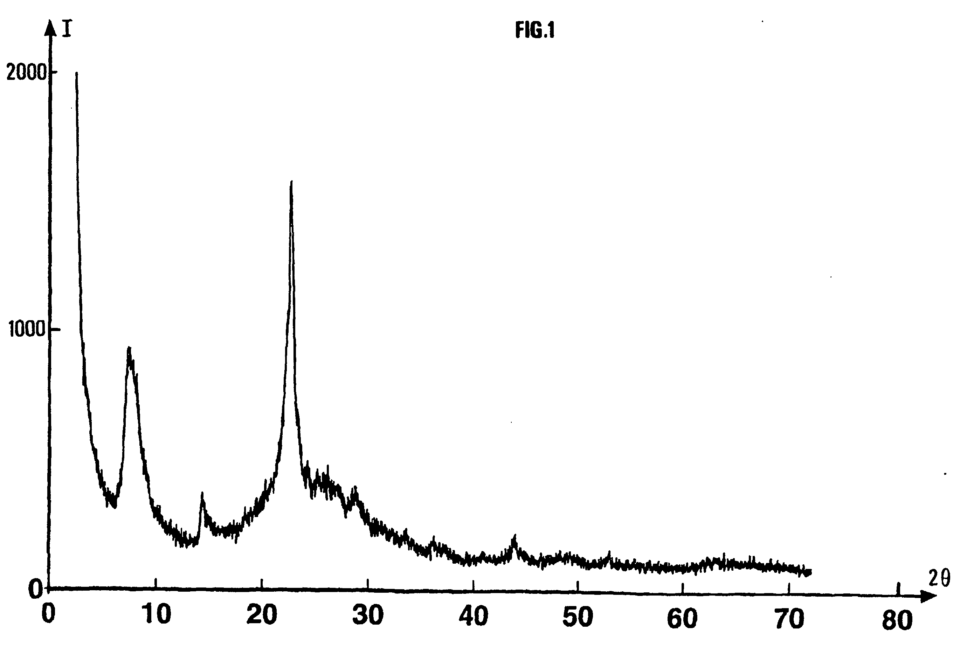

Le produit solide séché a été analysé par diffraction X de poudres et identifié comme étant de la zéolithe NU-88. Le diagramme obtenu est en conformité avec les résultats présentés au tableau 1. Le diffractogramme est donné dans la figure 1 [en ordonnée l'intensité I (unité arbitraire) et en abscisse 2θ (Cu K alpha)].

### Exemple 2 : Synthèse de zéolithe NU-88 avec une structure pentane-1,5-bis(méthylpyrrolidinium) bromure (PentPyrr).

La structure du pentane-1,5-bis(méthylpyrrolidinium) bromure est la suivante : 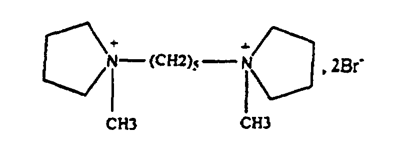 Un mélange réactionnel de composition molaire
60 SiO₂ : 1,714 Al₂O₃ : 12 Na₂O : 10 PentPyrr : 3000 H₂O
a été préparé à partir de
36,05 g de "CAB-O-SIL" (BDH Ltd)
7,908 g de solution SoAl 235 (Laroche)
   (composition en % poids : 22,10 % Al₂O₃; 19,80 % Na₂O; 58,10 % H₂O)
7,6 g de pastilles d'hydroxyde de sodium
41,2 g de PentPyrr (composition en % poids : 97,02 % PentPyrr; 2,98 % H₂O)
532 g d'eau distillée.

Le mélange a été préparé selon le mode opératoire suivant :
A - solution contenant l'hydroxyde de sodium et de l'aluminate de sodium dans de l'eau (approximativement 150 g)
B - solution contenant le PentPyrr dans de l'eau (approximativement 100 g)
C - dispersion du CAB-O-SIL dans l'eau restante.

La solution A a été ajoutée à la dispersion C sous agitation; on a ensuite ajouté la solution B. L'agitation a été poursuivie jusqu'à obtention d'un gel homogène. Le mélange obtenu a ensuite été transféré dans un autoclave en acier inoxydable d'une capacité de 1 litre. Le mélange a été porté à une température de 160°C. Cette température a été maintenue pendant toute la durée de la réaction. De plus, le mélange a été maintenu sous agitation à l'aide d'un agitateur à palettes inclinées.

Des échantillons du mélange réactionnel ont été régulièrement prélevés et le déroulement de la réaction a été surveillé par l'intermédiaire du pH. Après 22 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'émission atomique. On a trouvé la composition molaire suivante :

100 SiO₂ : 4,61 Al₂O₃ : 0,32 Na₂O.

Le produit solide séché a été analysé par diffraction X de poudres et identifié comme étant principalement constitué de zéolithe NU-88, avec des traces d'analcime (< 5%); le diagramme obtenu est conforme aux résultats présentés au tableau 1.

### Exemple 3 : Synthèse de la zéolithe NU-88 avec du PentPyrr

Un mélange réactionnel de composition molaire

60 SiO₂ : 1,714 Al₂O₃ : 18 Na₂O : 10 PentPyrr : 3000 H₂O

a été préparé à partir de
48,07 g de "CAB-O-SIL" (BDH Ltd)
10,587 g de solution SoAl 235 (composition en % poids : 22,01 % Al₂O₃; 19,81 % Na₂O; 58,18 % H₂O)
16,5 g de pastilles d'hydroxyde de sodium
55,3 g de PentPyrr ( composition en % poids : 97,02 % PentPyrr; 2,98 % d'eau)
708 g d'eau distillée.

Le mélange a été préparé selon le mode opératoire suivant :
A - solution contenant l'hydroxyde de sodium et de l'aluminate de sodium dans de l'eau (approximativement 200 g)
B - solution contenant le PentPyrr dans de l'eau (approximativement 150 g)
C - dispersion de CAB-O-SIL dans l'eau restante.

La solution A a été ajoutée à la dispersion C sous agitation; la solution B a ensuite été ajoutée. L'agitation a été poursuivie jusqu'à obtention d'un gel homogène. Le mélange obtenu a ensuite été transféré dans un autoclave en acier inoxydable d'une capacité de 1 litre. La température du mélange a été portée à 160°C. Cette température a été maintenue pendant toute la durée de la réaction. De plus, le mélange a été maintenu sous agitation à l'aide d'un agitateur à palettes inclinées.

Des échantillons du mélange réactionnel ont été régulièrement prélevés et le déroulement de la réaction a été surveillé par l'intermédiaire du pH. Après 15 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'émission atomique. On a trouvé la composition molaire suivante :

100 SiO₂ : 5,10 Al₂O₃ : 0,153 Na₂O.

Le produit solide séché a été analysé par diffraction X de poudres et identifié comme étant de la zéolithe NU-88; le diagramme obtenu est conforme aux résultats présentés au tableau 1.
L'analyse au microscope électronique à transmission a révélé que la morphologie dominante était caractérisée par des cristaux en forme de plaquettes liés entre eux de façon à former des agrégats. Ces plaquettes présentaient approximativement les dimensions suivantes : 100 x 100 x 10 nm.

### Exemple 4 : Synthèse de la zéolithe NU-88 avec du PentPyrr

On a reproduit l'expérience décrite dans l'exemple 3, à cette exception près que l'on a ajouté un germe de zéolithe NU-88 au mélange réactionnel; le poids du germe ajouté correspondait à 4 % en poids de la quantité totale de silice utilisée au cours de l'expérience.

Un mélange réactionnel de composition molaire

60 SiO₂ : 1,714 Al₂O₃ : 18 Na₂O : 10 PentPyrr : 3000 H₂O

a été préparé à partir de
36,05 g de "CAB-O-SIL" (BDH Ltd)
7,805 g de solution SoAl 235 (composition en % poids : 22,39 % Al₂O₃; 20,49 % Na₂O; 57,12 % H₂O)
12,3 g de pastilles d'hydroxyde de sodium
40,8 g de PentPyrr (composition en % poids : 97,99 % organique; 2,01 % H₂O)
532 g d'eau
1,44 g de NU-88 (sous la forme du produit préparé dans l'exemple 3).

Le mélange a été préparé selon le mode opératoire suivant :
A - solution contenant l'hydroxyde de sodium et de l'aluminate de sodium dans de l'eau (approximativement 150 g)
B - solution contenant le PentPyrr dans de l'eau (approximativement 100 g)
C - dispersion du germe de NU-88 dans de l'eau (approximativement 50 g)
D - dispersion du CAB-O-SIL dans l'eau restante.

La solution A a été ajoutée à la dispersion D sous agitation; la solution B a ensuite été ajoutée, puis la dispersion C. L'agitation a été poursuivie jusqu'à obtention d'un gel homogène. Le mélange obtenu a ensuite été transféré dans un autoclave en acier inoxydable d'une capacité de 1 litre. La température du mélange a été portée à 160°C. Cette température a été maintenue pendant toute la durée de la réaction. De plus, l'agitation a été maintenue au moyen d'un agitateur à palettes inclinées.

Des échantillons du mélange réactionnel ont été régulièrement prélevés et le déroulement de la réaction a été surveillé par l'intermédiaire du pH. Après 9 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'émission atomique. On a trouvé la composition molaire suivante :

100 SiO₂ : 7,0 Al₂O₃ : 1,26 Na₂O.

Le produit solide séché a été analysé par diffraction X de poudres et identifié comme étant principalement constitué de zéolithe NU-88, avec une faible quantité d'analcime (environ 5 %); le diagramme obtenu est conforme aux résultats présentés au tableau 1.

Il est évident que le fait d'ajouter le germe de NU-88 au mélange réactionnel a réduit la durée totale de préparation de la zéolithe NU-88.

### Exemple 5 : Synthèse de la zéolithe NU-88 avec de l'HexPyrr

Un mélange réactionnel de composition molaire

60 SiO₂ : 2 Al₂O₃ : 18 Na₂O : 20 HexPyrr : 5000 H₂O

a été préparé à partir de
28,16 g de "CAB-O-SIL" (BDH Ltd)
13,388 g de solution d'aluminate de sodium
   (composition en % poids : 11,90 % Al₂O₃; 23,07 % Na₂O; 65,02 % H₂O)
7,3 g de pastilles d'hydroxyde de sodium
68,3 g d'HexPyrr (composition en % poids : 94,75 % organique; 5,25 % H₂O)
689 g d'eau distillée.

Le mélange a été préparé selon le mode opératoire suivant :
A - solution contenant l'hydroxyde de sodium et l'aluminate de sodium dans de l'eau (approximativement 200 g)
B - solution contenant l'HexPyrr dans de l'eau (approximativement 150 g)
C - dispersion de CAB-O-SIL dans l'eau restante.

La solution A a été ajoutée à la dispersion C sous agitation; la solution B a ensuite été ajoutée. L'agitation a été poursuivie jusqu'à obtention d'un gel homogène. Le mélange obtenu a ensuite été transféré dans un autoclave en acier inoxydable d'une capacité de 1 litre. La température du mélange a été portée à 160°C. Cette température a été maintenue pendant toute la durée de la réaction. De plus, l'agitation du mélange a été maintenue au moyen d'un agitateur à palettes inclinées.

Des échantillons du mélange réactionnel ont été régulièrement prélevés et le déroulement de la réaction a été surveillé par l'intermédiaire du pH. Après 11 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'amission atomique. On a trouvé la composition molaire suivante :

100 SiO₂ : 5,4 Al₂O₃ : 2,64 Na₂O.

Le produit solide séché a été analysé par diffraction X de poudres et identifié comme étant un mélange de zéolithe NU-88 et d'analcime; la composition était approximativement la suivante : 85 % NU-88/15 % analcime.

### Exemple 6 : Synthèse de zéolithe NU-88 avec du PentPyrr

Un mélange réactionnel de composition molaire

60 SiO₂ : 1,50 Al₂O₃ : 18 Na₂O : 10 PentPyrr : 3000 H₂O

a été préparé à partir de
45,06 g de "CAB-O-SIL" (BDH Ltd)
15,931 g de solution d'aluminate de sodium
   (composition en % poids : 12,00 % Al₂O₃; 24,44 % Na₂O; 63,56 % H₂O)
13,0 g de pastilles d'hydroxyde de sodium
50,4 g de PentPyrr (composition en % poids : 99,26 % organique; 0,74 % H₂O)
662 g d'eau.

Le mélange a été préparé selon le mode opératoire suivant :
A - solution contenant l'hydroxyde de sodium et l'aluminate de sodium dans de l'eau (approximativement 200 g)
B - solution contenant le PentPyrr dans de l'eau (approximativement 150 g)
C - dispersion de CAB-O-SIL dans l'eau restante.

La solution A a été ajoutée à la dispersion C sous agitation; la solution B a ensuite été ajoutée. L'agitation a été poursuivie jusqu'à obtention d'un gel homogène. Le mélange obtenu a ensuite été transféré dans un autoclave en acier inoxydable d'une capacité de 1 litre. La température du mélange a été portée à 160°C. Cette température a été maintenue pendant toute la durée de la réaction. De plus, l'agitation a été maintenue au moyen d'un agitateur à palettes inclinées.

Des échantillons du mélange réactionnel ont été régulièrement prélevés et le déroulement de la réaction a été surveillé par l'intermédiaire du pH. Après 12 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'émission atomique. On a trouvé la composition molaire suivante :

100 SiO₂ : 4,7 Al₂O₃ : 0,20 Na₂O.

Le produit solide séché a été analysé par diffraction X de poudres et identifié comme étant de la zéolithe NU-88; le diagramme obtenu est conforme aux résultats présentés au tableau 1.

### Exemple 7 : Synthèse de zéolithe NU-88 avec de l'HexPyrr

Un mélange réactionnel de composition molaire

60 SiO₂ : 2 Al₂O₃ : 18 Na₂O : 10 HexPyrr : 3000 H₂O

a été préparé à partir de
36,05 g de "CAB-O-SIL"
15,309 g de solution d'aluminate de sodium
   (composition en % poids : 13,32 % Al₂O₃; 24,43 % Na₂O; 62,26 % H₂O)
9,6 g de pastilles d'hydroxyde de sodium
43,2 g d'HexPyrr (composition en % poids : 95,83 % organique; 4,17 % H₂O)
616 g d'eau distillée.

Le mélange a été préparé selon le mode opératoire suivant :
A - solution contenant l'hydroxyde de sodium et l'aluminate de sodium dans de l'eau (approximativement 200 g)
B - solution contenant l'HexPyrr dans de l'eau (approximativement 150 g)
C - dispersion du CAB-O-SIL dans l'eau restante.

La solution A a été ajoutée à la dispersion C sous agitation. La solution B a ensuite été ajoutée. L'agitation a été poursuivie jusqu'à obtention d'un gel homogène. Le mélange obtenu a ensuite été transféré dans un autoclave en acier inoxydable d'une capacité de 1 litre. La température du mélange a été portée à 160°C. Cette température a été maintenue pendant toute la durée de la réaction. De plus, l'agitation a été maintenue au moyen d'un agitateur à palettes inclinées.

Des échantillons du mélange réactionnel ont été régulièrement prélevés et le déroulement de la réaction a été surveillé par l'intermédiaire du pH. Après 12 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Al et Na dans le produit a été effectuée par spectroscopie d'émission atomique. On a trouvé la composition molaire suivante :

100 SiO₂ : 6,54 Al₂O₃ : 3,92 Na₂O.

Le produit solide séché a été analysé par diffraction X de poudres et identifié comme étant un mélange de zéolithe NU-88 et d'analcime; la composition était approximativement la suivante : 70 % NU-88/30 % analcime.

### Exemple 8 : Synthèse de NU-88 avec une structure butane-1,4-bis(methylpyrrolidinium) bromure (TetraPyrr)

La structure du butane-1,4-bis(methylpyrrolidinium) bromure est la suivante :

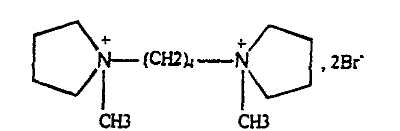

Un mélange réactionnel de composition molaire :

60 SiO₂ : 1.5 Al₂O₃ : 15 Na₂O : 10 TetraPyrr : 3000 H₂O

a été préparé à partir de
24,03g "CAB-O-SIL" (BDH Ltd)
9,711 g de solution d'aluminate de sodium (composition en % poids : 10,52 % Al₂O₃ ; 21,56% Na₂O ; 67,92% H₂O)
5,30 g de pastilles d'hydroxyde de sodium
25,81 g de TetraPyrr
   (composition en % poids : 99,25% TetraPyrr ; 0,25% H₂O))
352,45 g d'eau distillée.

Le mélange a été préparé selon le mode opératoire suivant :
A - solution contenant l'hydroxide de sodium et l'aluminate de sodium dans de l'eau (approximativement 120 g)
B - solution contenant leTetraPyrr dans de l'eau (approximativement 120 g)
C - dispersion du CAB-O-SIL dans l'eau restante.

La solution A a été ajoutée à la dispersion C, sous agitation ; on a ensuite ajouté la solution B. L'agitation a été poursuivie jusqu'à obtention d'un gel homogène. Le mélange obtenu a ensuite été transféré dans un autoclave en acier inoxydable d'une capacité de 1 litre. Le mélange a été porté à une température de 160° C. Cette température a été maintenue pendant toute la durée de la réaction. De plus le mélange a été maintenu sous agitation à 45 tours/minute dans un système rotatif.

Après 10 jours à 160° C, la température du mélange réactionnel a été brusquement abaissée à température ambiante et le produit a été évacué. La substance a été ensuite filtrée ; le produit solide a été lavé à l'eau déminéralisée et séché pendant plusieurs heures à 110°C.

L'analyse du Si, Al et Na dans le produit a été effectuée par spectroscopie d'émission atomique; on a trouvé la composition molaire suivante :

100 SiO₂ : 4,46 Al₂O₃ : 2,72 Na₂O

Le produit solide séché a été analysé par diffraction X de poudre et identifié comme étant de la zéolithe NU-88.

### Exemple 9 : Synthèse de NU-88 avec une structure butane-1,4-bis(bisquinuclidinium) bromure (TetraBisQ)

La structure du butane-1,4-bis(bisquinuclidinium) bromure est la suivante :

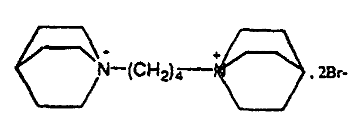

Un mélange réactionnel de composition molaire

60 SiO₂ : 1.5 Al₂O₃ : 10 Na₂O : 10 TetraBisQ : 3000 H₂O

a été préparé à partir de :
3,00 g "CAB-O-SIL" (BDH Ltd)
1,214 g de solution aluminate de sodium
   (composition en % poids : 10,52% Al₂O₃; 21,56% Na₂O ; 67,92% H₂O)
0,33 g de pastilles d'hydroxyde de sodium
3,85 g de TetraBisQ (composition en % poids : 94,8% TetraBisQ ; 5,2% H₂O))
43,94 g d'eau distillée.

Le mélange a été préparé selon le mode opératoire suivant :
A - solution contenant l'hydroxide de sodium et l'aluminate de sodium dans de l'eau (approximativement 11g)
B - solution contenant le Tetra-bisQ dans de l'eau (approximativement 11 g)
C - dispersion du CAB-O-SIL dans l'eau restant.

La solution A a été ajoutée à la dispersion C, sous agitation ; on a ensuite ajouté la solution B. L'agitation a été poursuivie jusqu'à obtention d'un gel homogène. Le mélange obtenu a ensuite été transféré dans un autoclave en acier inoxydable d'une capacité de 75 ml. Le mélange a été porté à une température de 180°C. Cette température a été maintenue pendant toute la durée de la réaction. De plus le mélange a été maintenu sous agitation à 45 tours/minute dans un système rotatif.

Après 12 jours à 180° C, la température du mélange réactionnel a été brusquement abaissée à température ambiante et le produit a été évacué. La substance a été ensuite filtrée ; le produit solide a été lavé à l'eau déminéralisée et séché pendant plusieurs heures à 110°C.

L'analyse du Si, Al et Na dans le produit a été effectuée par spectroscopie d'émission atomique; on a trouvé la composition molaire suivante :

100 SiO₂ : 3,1 Al₂O₃ : 0,65 Na₂O

Le produit solide séché a été analysé par diffraction X de poudre identifié comme étant de la zéolithe NU-88.

### Exemple 10 : Synthèse de Ga-NU-88

Un mélange réactionnel de composition

60 SiO₂ : 2 Ga 2O₃ : 18 Ma₂O : 10 HexPyrr : 3000 H₂O

a été préparé à partir de
36,05 g de "CAB-O-SIL" (BDH Ltd)
21,872 g de solution de gallate de sodium
   (composition en % poids : 17,14 % Ga₂O₃; 23,84 % Na₂O; 59,01 % H₂O)
7,7 g de pastilles d'hydroxyde de sodium
42,2 g d'HexPyrr (composition en % poids : 99,01 % organique; 1,99 % H₂O)
524 g d'eau.

Le mélange a été préparé selon le mode opératoire suivant :
A - solution contenant l'hydroxyde de sodium et le gallate de sodium dans de l'eau (approximativement 150 g)
B - solution contenant l'HexPyrr dans de l'eau (approximativement 100 g)
C - dispersion de CAB-O-SIL dans l'eau restante.

La solution A a été ajoutée à la dispersion C sous agitation; la solution B a ensuite été ajoutée. L'agitation a été poursuivie jusqu'à obtention d'un gel homogène. Le mélange obtenu a ensuite été transféré dans un autoclave en acier inoxydable d'une capacité de 1 litre. La température du mélange a été portée à 160°C. Cette température a été maintenue pendant toute la durée de la réaction. De plus, l'agitation a été maintenue au moyen d'un agitateur à palettes inclinées.

Des échantillons du mélange réactionnel ont été régulièrement prélevés et le déroulement de la réaction a été surveillé par l'intermédiaire du pH. Après 10 jours à 160°C, la température du mélange réactionnel a été brusquement abaissée à la température ambiante et le produit a été évacué. La substance a ensuite été filtrée; le produit solide obtenu a été lavé à l'eau déminéralisée et séché pendant plusieurs heures à 110°C.

L'analyse du Si, Ga et Na dans le produit a été effectuée par spectroscopie d'émission atomique. On a trouvé la composition molaire suivante :

100 SiO₂ : 5,35 Ga₂O₃ : 1,07 Na₂O.

Le produit solide séché a été analysé par diffraction X de poudres et identifié comme étant du Ga-NU-88; le diagramme obtenu est conforme aux résultats présentés au tableau 1.

### Exemple 11 : Préparation de H-NU-88

Une partie du produit de l'exemple 3 a été calcinée sous azote pendant 24 heures à 550°C; cette étape a été immédiatement suivie par une seconde calcination sous air, également à 550°C pendant 24 heures.

La substance obtenue a ensuite été mise en contact pendant 2 heures à température ambiante avec une solution aqueuse à 1 mole de chlorure d'ammonium en utilisant 50 ml de solution par gramme de produit calciné solide. La substance a ensuite été filtrée, lavée à l'eau permutée et séchée à 110°C. Ce traitement a été répété. La substance a ensuite été calcinée sous air pendant 24 heures à 550°C.

L'analyse du Si, Al et Na dans le produit, effectuée par spectroscopie d'émission atomique, a donné la composition molaire suivante :

100 SiO₂ : 4,05 Al₂O₃ : < 0,004 Na₂O.

### Exemple 12 : Préparation de H-NU-88

Une partie du produit de l'exemple 6 a été calcinée sous azote pendant 24 heures à 550°C; cette étape a été immédiatement suivie d'une seconde calcination sous air à 450°C, pendant 24 heures.

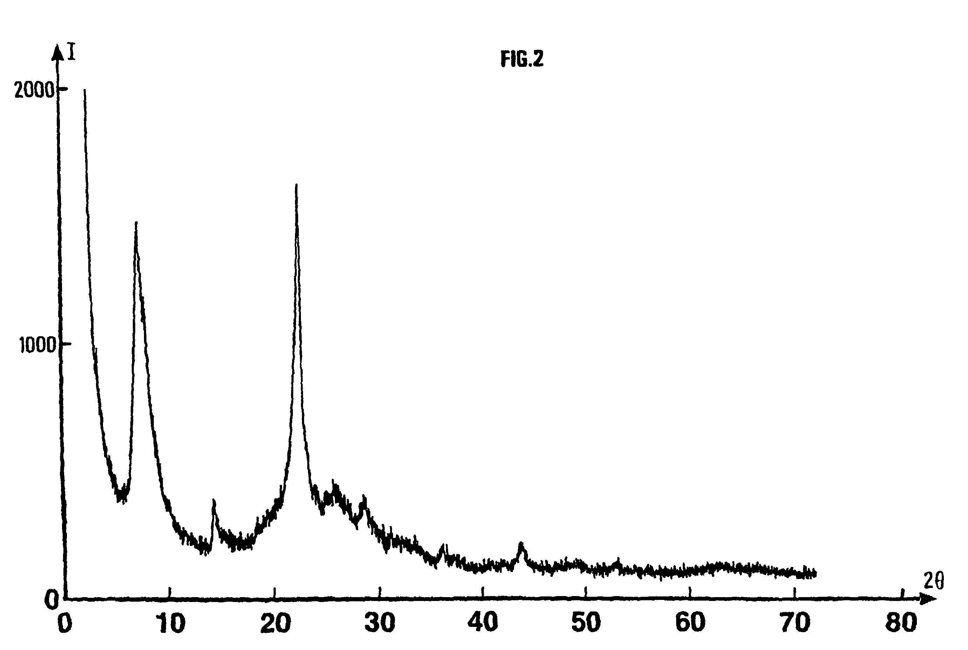

La substance obtenue a ensuite été mise en contact pendant 2 heures à température ambiante avec une solution aqueuse à 1 mole de chlorure d'ammonium en utilisant 50 ml de solution par gramme de produit calciné solide. La substance a ensuite été filtrée, lavée à l'eau permutée et séchée à 110°C. Ce traitement a été répété. La substance a ensuite été calcinée sous air pendant 24 heures, à 550°C. Le produit calciné a été analysé en diffraction des rayons X. Le diffractogramme obtenu est donné figure 2 [en abscisse 2θ (Cu K alpha) et en ordonnée l'intensité I (unité arbitraire)]. Le diagramme de diffraction X est en accord avec le tableau 2.

L'analyse du Si, Al et Na dans le produit, effectuée par spectroscopie d'émission atomique, a donné la composition molaire suivante :

100 SiO₂ : 3,80 Al₂O₃ : 0,011 Na₂O

### Exemple 13 : Evaluation des propriétés catalytiques de la zéolithe H-NU-88 en craquage du méthylcyclohexane

1,2 g de zéolithe H-NU-88, préparée dans l'exemple 11, sont introduits dans un réacteur à lit fixe, tubulaire. La température du réacteur est portée à 500°C, puis le cyclohexane est introduit dans le réacteur. Le gaz diluant utilisé est de l'azote et le rapport molaire N2/cyclohexane admis dans le réacteur est de 12. La vitesse spatiale de méthylcyclohexane, c'est à dire la masse de méthylcyclohexane utilisé par par unité de masse de zéolithe H-NU-88 et par unité de temps est telle qu'elle permette d'obtenir une conversion de 60% poids. Les sélectivités pour les différents produits obtenus sont regroupés dans le tableau ci-après.

| Composés | Sélectivités (% poids) |
|---|---|
| Gaz (C1+C2+C3+C4) oléfines et paraffines | 46,7 |
| Composés C5-C6 | 11,5 |
| Composés en C7 isomères du méthylcyclohexane | 2,6 |
| Toluène + C8+ (1) | 39,2 |

| | |
|---|---|
| (1) Composés comportant au moins 8 atomes de carbone | |

Cet exempte montre que la zéolithe H-NU-88 est suffisamment active pour conduire au craquage du méthylcyclohexane et une sélectivités en gaz (C1-C4) de 46,7 % poids pour une conversion de 60% poids.

## Revendications

1. Zéolithe **caractérisée par** :
-i) une composition chimique exprimée sur une base anhydre, en termes de rapports molaires d'oxydes, par la formule :
100 XO₂, m Y₂O₃, p R_{2/n}O,
où
m est égal ou inférieur à 10,
p est égal ou inférieur à 20,
R représente un ou plusieurs cations de valence n,
X est le silicium et/ou le germanium,
Y est choisi dans le groupe formé par les éléments suivants : l'aluminium, le fer, le gallium, le bore, le titane, le vanadium, le zirconium, le molybdène, l'arsenic, l'antimoine, le chrome et le manganèse, et
-ii) le fait qu'elle présente, sous forme brute de synthèse, un diagramme de diffraction X comportant les résultats présentés dans le tableau 1 suivant :
**Tableau 1:**
| Tableau de diffraction des rayons X de la zéolithe NU-88 (brute de synthèse) | |
|---|---|
| dₕₖₗ (10⁻¹⁰m) | l/lₘₐₓ |
| 12,1±0,35 | F ou TF (1) |
| 11,0±0,30 | F(1) |
| 9,88±0,25 | m (1) |
| 6,17±0,15 | f |
| 3,97±0,09 | TF (2) |
| 3,90±0,08 | TF (2) |
| 3,80±0,08 | f (2) |
| 3,66±0,07 | tf |
| 3,52±0,07 | tf |
| 3,27±0,07 | tf |
| 3,09±0,06 | f |
| 2,91±0,06 | f |
| 2,68±0,06 | tf |
| 2,49±0,05 | tf |
| 2,20±0,05 | tf |
| 2,059±0,05 | f |
| 1,729±0,04 | tf |
| | |
|---|---|
| (1) Ces pics ne sont pas résolus et font partie d'un même massif. | |
| (2) Ces pics ne sont pas résolus et font partie du même massif. | |

2. Zéolithe selon la revendication 1 telle que m est compris entre 0,6 et 8 et p est compris entre 0 et 3,92.

3. Zéolithe **caractérisée par** :
i) une composition chimique exprimée sur une base anhydre, en termes de rapports molaires d'oxydes, par la formule
100 XO₂ : inférieur ou égal à 10 Y₂O₃ : inférieur ou égal à 10 M₂O,
où
X est le silicium et/ou le germanium,
Y est choisi dans le groupe formé par les éléments suivants : l'aluminium, le fer, le gallium, le bore, le titane, le vanadium, le zirconium, le molybdène, l'arsenic, l'antimoine, le chrome et le manganèse, et
M est au moins un cation de métal alcalin (groupe lA de la Classification Périodique des Eléments) et/ou l'ammonium et/ou l'hydrogène,
ii) le fait qu'elle présente, sous forme calcinée, un diagramme de diffraction X comportant les résultats présentés dans le tableau 2 suivant :
**Tableau 2 :**
| Tableau de diffraction des rayons X de la zéolithe NU-88 (forme hydrogène) | |
|---|---|
| dₕₖₗ (10⁻¹⁰m) | l/lₘₐₓ |
| 12,1±0,35 | TF(1) |
| 11,0±0,30 | F ou TF (1) |
| 9,92±0,25 | f ou m (1) |
| 8,83±0,20 | tf |
| 6,17±0,15 | f |
| 3,99±0,10 | F ou TF (2) |
| 3,91±0,08 | TF (2) |
| 3,79±0,08 | f ou m (2) |
| 3,67±0,07 | tf |
| 3,52±0,07 | tf |
| 3,09±0,06 | f |
| 2,90±0,06 | f |
| 2,48±0,05 | f |
| 2,065±0,05 | f |
| 1,885±0,04 | tf |
| 1,733±0,04 | tf |
| | |
|---|---|
| (1) Ces pics ne sont pas résolus et font partie d'un même massif. | |
| (2) Ces pics ne sont pas résolus et font partie du même massif. | |

4. Zéolithe selon l'une des revendications 1 ou 2 ayant la composition chimique exprimée sur une base anhydre, en termes de rapports molaires d'oxydes suivante :
100 XO₂ : inférieur ou égal à 10 Y₂O₃ : inférieur ou égal à 10 Q : inférieur ou égal à 10 M₂O,
X est le silicium et/ou le germanium,
Y est choisi dans le groupe formé par les éléments suivants : l'aluminium, le fer, le gallium, le bore, le titane, le vanadium, le zirconium, le molybdène, l'arsenic, l'antimoine, le chrome et le manganèse, et
où M est au moins un cation de métal alcalin (groupe lA de la Classification Périodique des Eléments) et/ou l'ammonium, et Q est au moins un cation organique azoté ou un précurseur de cation organique azoté ou un produit de décomposition de cation organique azoté.

5. Zéolithe selon la revendication 4 telle que :
Q est un cation de bis(méthyl pyrrolidinium), ou un de ses produits de décomposition ou un de ses précurseurs, de formule générale 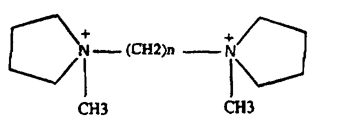 avec n = 4, 5 ou 6, ou Q est le butane-1,4-bis(bisquinuclidinium) bromure, ou un de ses produits de décomposition ou un de ses précurseurs, de formule chimique suivante :

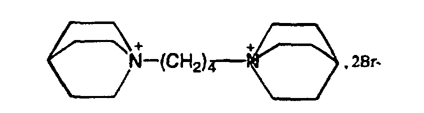

6. Zéolithe selon l'une des revendications 4 ou 5 telle que Q est l'hexane-1,6-bis(méthylpyrrolidinium) bromure, le pentane-1,5-bis(méthylpyrrolidinium) bromure, le butane-1,4- bis(méthylpyrrolidinium) bromure ou le butane-1,4-bis(quinuclidinium) bromure.

7. Zéolithe selon l'une des revendications 1 à 6 telle que X est le silicium et Y est l'aluminium.

8. Zéolithe selon l'une des revendication 1, 2, 4, 5, 6 ou 7 au moins en partie sous forme H+ ou NH4+ ou métallique, ledit métal étant choisi dans le groupe formé par les groupes IA, IB, IIA, IIB, IIIA, IIIB (y compris les terres rares), VIII, Sn, Pb et Si.

9. Catalyseur comprenant une zéolithe selon l'une des revendications 1 à 8.

10. Catalyseur selon la revendication 9 comprenant en outre un liant ou un support ou une autre zéolithe ou un métal choisi dans le groupe formé par les éléments Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Re et Rh.

11. Procédé de conversion d'hydrocarbures utilisant un catalyseur selon l'une des revendications 9 ou 10.

12. Procédé de conversion d'hydrocarbures selon la revendication 11 au cours duquel on met en contact un alkylbenzène ou un mélange d'alkylbenzènes dans des conditions d'isomérisation, en phase vapeur ou liquide, avec un catalyseur contenant de la zéolithe NU-88.

13. Procédé d'isomérisation ou d'hydroisomérisation du xylène selon la revendication 12.

14. Procédé de conversion d'hydrocarbures selon la revendication 11 dans lequel on réalise l'isomérisation et l'hydro-isomérisation de paraffines normales comprenant de 4 à 10 atomes de carbone par molécule.

15. Procédé de conversion d'hydrocarbures selon la revendication 11 dans lequel on réalise l'isomérisation d'oléfines.

16. Procédé de conversion d'hydrocarbures selon la revendication 11 dans lequel on réalise le déparaffinage catalytique.

17. Procédé de conversion d'hydrocarbures selon la revendication 11 dans lequel on met en contact un ou plusieurs composés aromatiques alkylés dans des conditions de transalkylation, en phase vapeur ou en phase liquide, avec un catalyseur contenant de la zéolithe NU-88.

18. Procédé de transalkylation et/ou de dismutation des xylènes selon la revendication 17.

19. Procédé de dismutation et/ou de transalkylation du toluène et de composés aromatiques comprenant au moins 9 atomes de carbone par molécule selon la revendication 17.

20. Procédé de conversion d'hydrocarbures selon la revendication 11 dans lequel on met en contact un composé oléfinique ou aromatique avec un composé alkylant approprié dans des conditions d'alkylation, en phase vapeur ou en phase liquide, avec un catalyseur contenant de la zéolithe NU-88.

21. Procédé de conversion d'hydrocarbures selon la revendication 11 dans lequel on conduit des réactions d'oligomérisation, de cyclisation et/ou d'aromatisation sur des composés insaturés ou sur des composés saturés ou sur des mélanges d'hydrocarbures.

22. Procédé de conversion d'hydrocarbures selon la revendication 11 dans lequel on conduit des réactions de craquage.

23. Procédé de conversion d'hydrocarbures selon la revendication 11 dans lequel on réalise des réactions d'hydrogénation et/ou de déshydrogénation.

24. Utilisation d'un catalyseur selon l'une des revendications 9 ou 10 dans laquelle on conduit une formation d'éthers.

25. Utilisation d'un catalyseur selon l'une des revendications 9 ou 10 dans laquelle on conduit l'élimination catalytique des oxydes d'azote.

26. Utilisation selon la revendication 25 dans lequel l'élimination est conduite par réduction.

27. Utilisation selon l'une des revendications 25 ou 26 dans lequel l'élimination est conduite par réduction par des composés azotés ou par des hydrocarbures.

## Patentansprüche

1. Zeolith, **dadurch gekennzeichnet, dass** er aufweist:
i) eine chemische Zusammensetzung, ausgedrückt durch die Oxid-Molverhältnisse auf wasserfreier Basis, der Formel
100 XO₂, m Y₂O₃, p R_{2/n}O,
worin bedeuten:
m eine Zahl von ≤ 10,
p eine Zahl von ≤ 20,
R ein oder mehrere Kationen mit der Valenz n,
X Silicium und/oder Germanium,
Y ein Element, ausgewählt aus der Gruppe der folgenden Elemente:
Aluminium, Eisen, Gallium, Bor, Titan, Vanadin, Zirkonium, Molybdän, Arsen, Antimon, Chrom und Mangan, und
ii) als Synthese-Rohprodukt ein Röntgenbeugungsdiagramm, das die in der folgenden Tabelle 1 angegebenen Ergebnisse umfasst:
**Tabelle 1 :**
| Röntgenbeugungsdiagramm von Zeolith NU-88 (Synthese-Rohprodukt) | |
|---|---|
| dₕₖₗ (10⁻¹⁰m) | l/lₘₐₓ |
| 12,1 ±0,35 11,0 ± 0,30 | F oder TF ⁽¹⁾ F ⁽¹⁾ |
| 9,88 ± 0,25 6,17±0,15 | m ⁽¹⁾ f |
| 3,97 ± 0,09 3,90 ± 0,08 | TF ⁽²⁾ TF ⁽²⁾ |
| 3,80 ± 0,08 3,66 ± 0,07 | f ⁽²⁾ tf |
| 3,52 ± 0,07 3,27 ± 0,07 | tf tf |
| 3,09 ± 0,06 2,91 ± 0,06 | f f |
| 2,68 ± 0,06 2,49 ± 0,05 | tf tf |
| 2,20 ± 0,05 2,059 ± 0,05 | tf f |
| 1,729 ± 0,04 | tf |
| | |
|---|---|
| ⁽¹⁾ diese Peaks sind nicht aufgelöst und Teil eines Gesamtpeaks | |
| ⁽²⁾ diese Peaks sind nicht aufgelöst und Teil eines Gesamtpeaks | |

2. Zeolith nach Anspruch 1, **dadurch gekennzeichnet, dass** m zwischen 0,6 und 8 und p zwischen 0 und 3,92 liegen.

3. Zeolith, **dadurch gekennzeichnet, dass** er aufweist:
i) eine chemische Zusammensetzung, ausgedrückt durch die Oxid-Molverhältnisse auf wasserfreier Basis, der Formel
100 XO₂: ≤ 10 Y₂O₃: ≤ 10 M₂O,
worin bedeuten:
X Silicium und/oder Germanium,
Y ein Element, ausgewählt aus der folgenden Gruppe von Elementen: Aluminium, Eisen, Gallium, Bor, Titan, Vanadin, Zirkonium, Molybdän, Arsen, Antimon, Chrom und Mangan, und
M mindestens ein Kation eines Alkalimetalls (der Gruppe IA des Periodischen Systems der Elemente) und/oder Ammonium und/oder Wasserstoff, und
ii) in der calcinierten Form ein Röntgenbeugungsdiagramm, das die in der folgenden Tabelle 2 angegebenen Ergebnisse umfasst:
**Tabelle 2:**
| Röntgenbeugungsdiagramm des Zeolith NU-88 (in der Wasser stoffform) | |
|---|---|
| dₕₖₗ (10⁻¹⁰m) | I/Iₘₐₓ |
| 12,1 ± 0,35 11,0 ± 0,30 | TF ⁽¹⁾ F oder TF ⁽¹⁾ |
| 9,92 ± 0,25 8,83 ± 0,20 | f oder m ⁽¹⁾ tf |
| 6,17 ± 0,15 3,99 ± 0,10 | f F oder TF ⁽²⁾ |
| 3,91 ± 0,08 3,79 ± 0,08 | TF⁽²⁾ f oder m ⁽²⁾ |
| 3,67 ± 0,07 3,52 ± 0,07 | tf tf |
| 3,09 ± 0,06 2,90 ± 0,06 | f f |
| 2,48 ± 0,05 2,065 ± 0,05 | f f |
| 1,885 ± 0,04 1,733 ± 0,04 | tf tf |
| | |
|---|---|
| ⁽¹⁾ diese Peaks sind nicht aufgelöst und Teil eines Gesamtpeaks | |
| ⁽²⁾ diese Peaks sind nicht aufgelöst und Teil eines Gesamtpeaks | |

4. Zeolith nach einem der Ansprüche 1 oder 2, der die folgende chemische Zusammensetzung, ausgedrückt durch die Oxid-Molverhältnisse auf wasserfreier Basis, aufweist:
100 XO₂: ≤ 10 Y₂O₃: ≤ 10 Q: ≤ 10 M₂O
worin bedeuten:
X Silicium und/oder Germanium,
Y ein Element, ausgewählt aus der Gruppe der folgenden Elemente: Aluminium, Eisen, Gallium, Bor, Titan, Vanadin, Zirkonium, Molybdän, Arsen, Antimon, Chrom und Mangan,
M mindestens ein Kation eines Alkalimetalls (Gruppe IA des Periodischen Systems der Elemente) und/oder Ammonium und
Q mindestens ein stickstoffhaltiges organisches Kation oder einen Vorläufer eines stickstoffhaltigen organischen Kations oder ein Zersetzungsprodukt eines stickstoffhaltigen organischen Kations.

5. Zeolith nach Anspruch 4, worin bedeuten:
Q ein Bis(methylpyrrolidinium)-Kation oder eines seiner Zersetzungsprodukte oder eines seiner Vorläufer der allgemeinen Formel 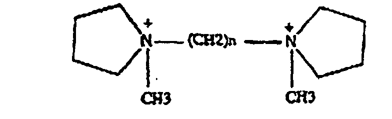 worin n = 4, 5 oder 6 oder
Q für Butan-1,4-bis(bischinuclidinium)-bromid oder eines seiner Zersetzungsprodukte oder einen seiner Vorläufer der folgenden Formel steht

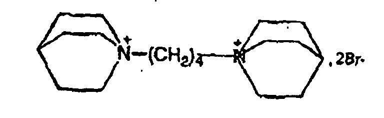

6. Zeolith nach einem der Ansprüche 4 oder 5, worin Q für Hexan-1,6-bis(methylpyrrolidinium)bromid, Pentan-1,5-bis(methylpyrrolidinium)-bromid, Butan-1,4-bis(methylpyrrolidinium)-bromid oder Butan-1,4-bis(chinuclidinium)-bromid steht.

7. Zeolith nach einem der Ansprüche 1 bis 6, worin X für Silicium und Y für Aluminium stehen.

8. Zeolith nach einem der Ansprüche 1, 2, 4, 5, 6 oder 7, der mindestens zum Teil in der H⁺- oder NH₄⁺- oder metallischen Form vorliegt, wobei das genannte Metall ausgewählt ist aus der Gruppe, die gebildet wird durch die Gruppen IA, IB, IIA, IIB, IIIA, IIIB (einschließlich der Seltenen Erden), VIII, Sn, Pb und Si.

9. Katalysator, der einen Zeolith nach einem der Ansprüche 1 bis 8 umfasst.

10. Katalysator nach Anspruch 9, der außerdem ein Bindemittel oder einen Träger oder einen anderen (weiteren) Zeolith oder ein Metall, ausgewählt aus der Gruppe der Elemente Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Re und Rh, umfasst.

11. Verfahren zur Umwandlung von Kohlenwasserstoffen, in dem ein Katalysator nach einem der Ansprüche 9 oder 10 verwendet wird.

12. Verfahren zur Umwandlung von Kohlenwasserstoffen nach Anspruch 11, in dessen Verlauf ein Alkylbenzol oder ein Gemisch von Alkylbenzolen unter Isomerisierungs-Bedingungen in der Gasphase (Dampfphase) oder in flüssiger Phase mit einem den Zeolith NU-88 enthaltenden Katalysator in Kontakt gebracht wird.

13. Verfahren zur Isomerisierung oder Hydroisomerisierung von Xylol nach Anspruch 12.

14. Verfahren zur Umwandlung von Kohlenwasserstoffen nach Anspruch 11, in dem eine Isomerisierung und Hydroisomerisierung von n-Paraffinen mit 4 bis 10 Kohlenstoffatomen pro Molekül durchgeführt wird.

15. Verfahren zur Umwandlung von Kohlenwasserstoffen nach Anspruch 11, in dem eine Isomerisierung von Olefinen durchgeführt wird.

16. Verfahren zur Umwandlung von Kohlenwasserstoffen nach Anspruch 11, in dem eine katalytische Entparaffinierung durchgeführt wird.

17. Verfahren zur Umwandlung von Kohlenwasserstoffen nach Anspruch 11, in dem eine oder mehrere alkylierte aromatische Verbindungen unter Transalkylierungs-Bedingungen in der Gasphase (Dampfphase) oder in flüssiger Phase mit einem den Zeolith NU-88 enthaltenden Katalysator in Kontakt gebracht wird (werden).

18. Verfahren zur Transalkylierung und/oder Dismutation von Xylolen nach Anspruch 17.

19. Verfahren zur Dismutation und/oder Transalkylierung von Toluol und aromatischen Verbindungen, die mindestens 9 Kohlenstoffatome pro Molekül enthalten, nach Anspruch 17.

20. Verfahren zur Umwandlung von Kohlenwasserstoffen nach Anspruch 11, bei dem eine Olefin-Verbindung oder eine aromatische Verbindung mit einer geeigneten Alkylierungs-Verbindung unter Alkylierungs-Bedingungen in der Gasphase (Dampfphase) oder in flüssiger Phase mit einem den Zeolith NU-88 enthaltenden Katalysator in Kontakt gebracht wird.

21. Verfahren zur Umwandlung von Kohlenwasserstoffen nach Anspruch 11, in dem ungesättigte Verbindungen oder gesättigte Verbindungen oder Kohlenwasserstoff-Gemische Oligomerisations-, Cyclisierungs- und/oder Aromatisierungs-Reaktionen unterworfen werden.

22. Verfahren zur Umwandlung von Kohlenwasserstoffen nach Anspruch 11, in dem Crackungsreaktionen durchgeführt werden.

23. Verfahren zur Umwandlung von Kohlenwasserstoffen nach Anspruch 11, in dem Hydrierungs- und/oder Dehydrierungsreaktionen durchgeführt werden.

24. Verwendung eines Katalysators nach einem der Ansprüche 9 oder 10, bei der eine Etherbildung durchgeführt wird.

25. Verwendung eines Katalysators nach einem der Ansprüche 9 oder 10, bei der eine katalytische Eliminierung von Stickstoffoxiden durchgeführt wird.

26. Verwendung nach Anspruch 25, bei der die Eliminierung durch Reduktion durchgeführt wird.

27. Verwendung nach einem der Ansprüche 25 oder 26, bei der die Eliminierung durch Reduktion mit stickstoffhaltigen Verbindungen oder mit Kohlenwasserstoffen durchgeführt wird.

## Claims

1. A zeolite, **characterized by**:
i) a chemical composition with the following formula, expressed in terms of the mole ratios of the oxides for the anhydrous state:
100 XO₂, mY₂O₃, pR_{2/n}O
where
m is 10 or less;
p is 20 or less;
R represents one or more cations with valency n;
X represents silicon and/or germanium;
Y is selected from the group formed by the following elements: aluminium, iron, gallium, boron, titanium, vanadium, zirconium, molybdenum, arsenic, antimony, chromium and manganese; and
ii) an X ray diffraction diagram, in its as synthesized state, which comprises the results shown in Table 1:
**TABLE 1:**
| **X ray diffraction diagram for NU-88 zeolite (as synthesized state)** | |
|---|---|
| dₕₖₗ (10⁻¹⁰ m) | I/Iₘₐₓ |
| 12.1±0.35 11.0±0.30 | s or vs (1) s (1) |
| 9.88±0.25 6.17±0.15 | m (1) w |
| 3.97±0.09 3.90±0.08 | vs (2) vs (2) |
| 3.80±0.08 3.66±0.07 | w (2) vw |
| 3.52±0.07 3.27±0.07 | vw vw |
| 3.09±0.06 2.91±0.06 | w w |
| 2.68±0.06 2.49±0.05 | vw vw |
| 2.20±0.05 2.059±0.05 | vw w |
| 1.729±0.04 | vw |
| | |
|---|---|
| (1) These peaks were not resolved and formed part of a feature. | |
| (2) these peaks were not resolved and formed part of the same feature. | |

2. A zeolite according to claim 1, in which m is in the range 0.6 to 8 and p is in the range 0 to 3.92.

3. A zeolite **characterized by**:
i) a chemical composition with the following formula, expressed in terms of the mole ratios of the oxides for the anhydrous state:
100 XO₂: 10 or less Y₂O₃: 10 or less M₂O,
where
X represents silicon and/or germanium;
Y is selected from the group formed by the following elements: aluminium, iron, gallium, boron, titanium, vanadium, zirconium, molybdenum, arsenic, antimony, chromium and manganese; and
M is at least one alkali metal cation (group IA of the periodic table) and/or ammonium and/or hydrogen;
ii) an X ray diffraction diagram, in its calcined state, which comprises the results shown in Table 2:
**TABLE 2:**
| **X ray diffraction diagram for NU-88 zeolite (hydrogen form)** | |
|---|---|
| dₕₖₗ (10⁻¹⁰ m) | I/Iₘₐₓ |
| 12.1±0.35 11.0±0.30 | vs (1) s or vs (1) |
| 9.92±0.25 8.83±0.20 | w or m (1) vw |
| 6.17±0.15 3.99±0.10 | w s or vs (2) |
| 3.91±0.08 3.79±0.08 | vs (2) w or m (2) |
| 3.67±0.07 3.52±0.07 | vw vw |
| 3.09±0.06 2.90±0.06 | w w |
| 2.48±0.05 2.065±0.05 | w w |
| 1.885±0.04 1.733±0.04 | vw vw |
| | |
|---|---|
| (1) These peaks were not resolved and formed part of a feature. | |
| (2) these peaks were not resolved and formed part of the same feature. | |

4. A zeolite according to claim 1 or claim 2 with the following formula, expressed in terms of the mole ratios of the oxides for the anhydrous state:
100 XO₂: 10 or less Y₂O₃: 10 or less Q: 10 or less M₂O,
where
X represents silicon and/or germanium;
Y is selected from the group formed by the following elements: aluminium, iron, gallium, boron, titanium, vanadium, zirconium, molybdenum, arsenic, antimony, chromium and manganese;
M is at least one alkali metal cation (group IA of the periodic table) and/or ammonium; and
Q is at least one organic nitrogen-containing cation or a precursor of an organic nitrogen-containing cation or a decomposition product of an organic nitrogen-containing cation.

5. A zeolite according to claim 4, in which:
Q is a bis (methylpyrrolidinium) cation or a decomposition product thereof or a precursor thereof, with general formula: 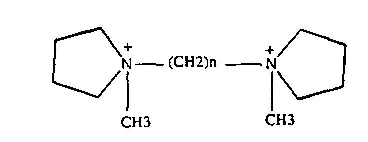
where n = 4, 5 or 6, or Q is butane-1,4-bis(bisquinuclidinium) bromide, or a decomposition product thereof or a precursor thereof, with the following chemical formula:

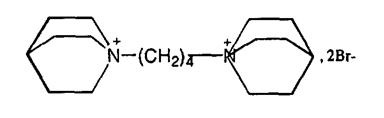

6. A zeolite according to claim 4 or claim 5, in which Q is hexane-1,6-bis(methylpyrrolidinium) bromide, pentane-1,5-bis(methylpyrrolidinium) bromide, butane-1,4-bis(methylpyrrolidinium) bromide or butane-1,4-bis(quinuclidinium) bromide.

7. A zeolite according to any one of claims 1 to 6, in which X is silicon and Y is aluminium.

8. A zeolite according to any one of claims 1, 2, 4, 5, 6 or 7, at least partially in its H⁺ form or in its NH₄⁺ form or in its metal form, said metal being selected from the group formed by groups IA, IB, IIA, IIB, IIIA, IIIB (including the rare earths), VIII, Sn, Pb and Si.

9. A catalyst comprising a zeolite according to any one of claims 1 to 8.

10. A catalyst according to claim 9, further comprising a binder or at least one support or at least one other zeolite or at least one metal selected from the group formed by the elements Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Re and Rh.

11. A hydrocarbon conversion process using a catalyst in accordance with claim 9 or 10.

12. A hydrocarbon conversion process according to claim 11, during which an alkylbenzene or a mixture of alkylbenzenes is brought into contact with a catalyst containing NU-88 zeolite under isomerisation conditions, in the vapour or liquid phase.

13. A process for the isomerisation or hydroisomerisation of xylene in accordance with claim 12.

14. A hydrocarbon conversion process according to claim 11, in which isomerisation and hydroisomerisation of normal paraffins containing 4 to 10 carbon atoms per molecule is carried out.

15. A hydrocarbon conversion process according to claim 11, in which olefin isomerisation is carried out.

16. A hydrocarbon conversion process according to claim 11, in which catalytic dewaxing is carried out.

17. A hydrocarbon conversion process according to claim 11, in which one or more aromatic alkylated compounds is brought into contact with a catalyst containing NU-88 zeolite under transalkylation conditions, in the vapour or liquid phase.

18. A process for the transalkylation and/or dismutation of xylenes in accordance with claim 17.

19. A process for the dismutation and/or transalkylation of toluene and aromatic compounds containing at least 9 carbon atoms per molecule, in accordance with claim 17.

20. A hydrocarbon conversion process according to claim 11, in which an olefinic or aromatic compound is brought into contact with a suitable alkylating compound and with a catalyst containing NU-88 zeolite under alkylating conditions, in the vapour or in the liquid phase.

21. A hydrocarbon conversion process according to claim 11, in which oligomerisation, cyclisation and/or aromatisation reactions are carried out on unsaturated compounds or on saturated compounds or on mixtures of hydrocarbons.

22. A hydrocarbon conversion process according to claim 11, in which cracking reactions are carried out.

23. A hydrocarbon conversion process according to claim 11, in which hydrogenation and/or dehydrogenation reactions are carried out.

24. Use of a catalyst according to claim 9 or claim 10, for the formation of ethers.

25. Use of a catalyst according to claim 9 or claim 10, for the catalytic elimination of oxides of nitrogen.

26. Use according to claim 25 in which elimination is effected by reduction.

27. Use according to claim 25 or 26, in which elimination is effected by reduction of nitrogen-containing compounds or of hydrocarbons.
